(19) Europäisches Patentamt
European Patent Office
Office européen des brevets

(11) **EP 3 360 932 A1**

(12) **EUROPEAN PATENT APPLICATION**
published in accordance with Art. 153(4) EPC

(43) Date of publication:
**15.08.2018 Bulletin 2018/33**

(51) Int Cl.:
*C09C 1/48* (2006.01)  *A61K 8/19* (2006.01)
*A61K 8/25* (2006.01)  *A61Q 1/02* (2006.01)
*C09C 1/58* (2006.01)  *C09C 3/06* (2006.01)
*C09D 7/12* (2006.01)  *C09D 201/00* (2006.01)

(21) Application number: **16853255.4**

(22) Date of filing: **03.10.2016**

(86) International application number:
**PCT/JP2016/004446**

(87) International publication number:
**WO 2017/061098 (13.04.2017 Gazette 2017/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **09.10.2015 JP 2015200799**

(71) Applicant: **Nippon Sheet Glass Company, Limited
Tokyo 108-6321 (JP)**

(72) Inventors:
• **SHIMOKAWA, Kosei
Tokyo 108-6321 (JP)**
• **DOSHITA, Kazuhiro
Tokyo 108-6321 (JP)**

(74) Representative: **Cabinet Beau de Loménie
158, rue de l'Université
75340 Paris Cedex 07 (FR)**

(54) **CARBON BLACK-CONTAINING COMPOSITE PARTICLES AND METHOD FOR PRODUCING CARBON BLACK-CONTAINING COMPOSITE PARTICLES**

(57) Carbon black-containing composite particles of the present invention each include a silicon oxide and carbon black particles dispersed inside the silicon oxide. When a coating film containing the carbon black-containing composite particles dispersed in a transparent matrix, having a concentration of the carbon black particles of 1 mass% , and having a thickness of 25 $\mu$m to 40 $\mu$m is formed on a hiding-chart specified in JIS (Japanese Industrial Standards) K 5600-4-1: 1999, a difference $L_W^* - L_B^*$ obtained by subtracting lightness $L_B^*$ of the coating film formed on a black part of the hiding-chart from lightness $L_W^*$ of the coating film formed on a white part of the hiding-chart is 30 or less. Thus carbon black-containing composite particles advantageous in exhibiting a good hiding property and reducing an undertone can be provided.

EP 3 360 932 A1

**Description**

TECHNICAL FIELD

The present invention relates to carbon black-containing composite particles and a method for producing the carbon black-containing composite particles.

BACKGROUND ART

Carbon black is conventionally used as a black coloring pigment for various purposes such as plastic materials, cosmetics, and paints. An article containing carbon black can exhibit black with a tinge of reddish black or bluish black (what they call an undertone) depending on the raw material of the carbon black.

As a black coloring pigment allowing control of its undertone, Patent Literature 1 describes carbon black for black color in which the sum B (ppm) of the contents of chromium, cobalt, and nickel and the content R (ppm) of the iron element are in a predetermined relation.

Patent Literature 2 describes an aniline black-containing water-based ink composition for a ball-point pen. With the aniline black contained, the composition exhibits black with a bluish undertone.

Patent Literature 3 describes silica-coated coloring pigment particles in which coloring pigment particles are coated by silica. According to Patent Literature 3, the coloring pigment particles may be carbon black. The silica-coated coloring pigment particles are used for a paint for forming a visible light shielding film.

CITATION LIST

Patent Literature 1: JP 06-136287 A
Patent Literature 2: JP 2002-371219 A
Patent Literature 3: JP 2004-204175 A

SUMMARY OF INVENTION

Technical Problem

It is conceivable that carbon black particles whose aggregates have a small diameter are used for a coloring pigment in order to improve the property of hiding the substrate. However, since such carbon black particles have a relatively strong cohesive force, it is difficult to uniformly disperse them in a matrix such as a synthetic resin. Moreover, a reddish undertone sometimes increases in intensity due to the diameter of the aggregates of the carbon black particles dispersed in the matrix.

According to the technique described in Patent Literature 1, metals such as chromium, cobalt, and nickel are added to control the undertone. In this case, the amount of these metals to be added needs to be determined in view of the possibility that these metals may be introduced from a raw material or production facility in production of a black coloring pigment. Therefore, it is hard to say that the technique described in Patent Literature 1 is easy. The technique described in Patent Literature 2 uses aniline black, which is an organic pigment, and does not take into account any application other than use in a water-color ink composition for a roller ball pen. Patent Literature 3 fails to specifically discuss the lightness of a visible light shielding film formed with the paint for forming a visible light shielding film described in Patent Literature 3.

In view of such circumstances, the present invention provides carbon black-containing composite particles that can be free of chromium, cobalt, and nickel, having great versatility, and being advantageous in exhibiting a good hiding property.

Solution to Problem

The present invention provides carbon black-containing composite particles each including a silicon oxide and carbon black particles dispersed inside the silicon oxide, wherein
when a coating film containing the carbon black-containing composite particles dispersed in a transparent matrix, having a concentration of the carbon black particles of 1 mass%, and having a thickness of 25 $\mu$m to 40 $\mu$m is formed on a hiding-chart specified in JIS (Japanese Industrial Standards) K 5600-4-1: 1999, a difference $L_W{}^* - L_B{}^*$ obtained by subtracting lightness $L_B{}^*$ of the coating film formed on a black part of the hiding-chart from lightness $L_W{}^*$ of the coating film formed on a white part of the hiding-chart is 30 or less.

The present invention also provides a method for producing the carbon black-containing composite particles, including:

introducing a dispersion containing colloidal silicon oxide particles and carbon black particles dispersed in water and having a pH of 7 or more into a liquid containing a solvent to yield, in the liquid, aggregates of the colloidal silicon oxide particles in which the carbon black particles are dispersed, the solvent being a protic solvent having a relative permittivity of 30 or less at 20°C or an aprotic solvent having a relative permittivity of 40 or less at 20°C, the solvent having a solubility of 0.05 g/100 ml or more in 20°C water; and

subjecting the aggregates to at least one treatment selected from drying, heating, and pressurization to obtain the carbon black-containing composite particles insoluble in water.

Advantageous Effects of Invention

The above carbon black-containing composite particles can be free of chromium, cobalt, and nickel. In addition, the above carbon black-containing composite particles have great versatility and can be used for cosmetics, paints, and plastic materials. Moreover, since $L_W^* - L_B^*$ is 30 or less, the above carbon black-containing composite particles are advantageous in exhibiting a good hiding property. Furthermore, the above method can produce such carbon black-containing composite particles with relative ease.

DESCRIPTION OF EMBODIMENTS

Hereinafter, embodiments of the present invention will be described. The following description relates to examples of the present invention, and the present invention is not limited by these examples.

Carbon black-containing composite particles of the present invention are composite particles in which carbon black particles are dispersed inside a silicon oxide. The carbon black-containing composite particles are produced in such a manner that a coating film in which the carbon black-containing composite particles are dispersed has predetermined characteristics. Specifically, a coating film containing the carbon black-containing composite particles dispersed in a transparent matrix, having a concentration of the carbon black particles of 1 mass%, and having a thickness of 25 $\mu$m to 40 $\mu$m has the predetermined characteristics. When this coating film is formed on a hiding-chart specified in JIS K 5600-4-1: 1999, a difference $L_W^* - L_B^*$ obtained by subtracting the lightness $L_B^*$ of the coating film formed on the black part of the hiding-chart from the lightness $L_W^*$ of the coating film formed on the white part of the hiding-chart is 30 or less.

In the present specification, the term "transparent matrix" refers to a matrix which is transparent to a visible ray having a wavelength ranging from 380 nm to 780 nm. The transparent matrix is, for example, an acrylic resin, and Auto Clear Super manufactured by NIPPON PAINT Co., Ltd. can be used, for example.

The lightness $L_W^*$ and lightness $L_B^*$ each refer to a lightness index $L^*$ specified in the L*a*b* color system (CIE (International Commission on Illumination) 1976). $L_W^* - L_B^*$ is a measure of the substrate hiding property of the above coating film. The smaller $L_W^* - L_B^*$ is, the better the hiding property of the above coating film is. Since $L_W^* - L_B^*$ of the above coating film is 30 or less, the carbon black-containing composite particles are advantageous in exhibiting a good hiding property.

The carbon black-containing composite particles are desirably produced so that the chroma $C^*$ of the above coating film formed on the white part of the hiding-chart is 5 or less. The chroma $C^*$ is defined by the following equation using psychometric chroma coordinates a* and b* of the L*a*b* color system. It can be said that the smaller the chroma $C^*$ of the coating film formed on the white part of the hiding-chart is, the more advantageous the carbon black-containing composite particles are in reducing the undertone.

$$C^* = \{(a^*)^2 + (b^*)^2\}^{1/2}$$

It can be thought that appropriate adjustment of the dispersion state of the carbon black particles in the carbon black-containing composite particles enables the coating film in which the carbon black-containing composite particles are dispersed to have a good hiding property and can reduce the undertone advantageously.

Chromium, cobalt, and nickel can be causative agents of an allergic reaction. Therefore, the carbon black-containing composite particles desirably do not contain chromium, cobalt, and nickel.

The carbon black-containing composite particles, for example, do not contain an organic pigment such as aniline black. This increases the versatility of the carbon black-containing composite particles.

The carbon black-containing composite particles each have the shape of a sphere or flake. The term "shape of a sphere" as used herein refers to a shape in which the ratio of the maximum diameter to the minimum diameter is within the range of 1.0 to 1.5. The term "shape of a flake" refers to the shape of a plate whose principal surfaces can be regarded as flat or curved surfaces and in which the ratio of the diameter of each principal surface to the thickness is 3 or more. The term "diameter of a principal surface" refers to the diameter of a circle having an area equal to that of the principal surface.

In the case where the carbon black-containing composite particles have the shape of a sphere, the average particle diameter of the carbon black-containing composite particles is, for example, 1 $\mu$m to 30 $\mu$m, desirably 2 $\mu$m to 15 $\mu$m, and more desirably 2 $\mu$m to 10 $\mu$m. Thus, the coating film in which the carbon black-containing composite particles are dispersed has a good hiding property more reliably. In the present specification, the term "average particle diameter of the carbon black-containing composite particles" refers to the particle diameter corresponding to a cumulative volume of 50% in the volume-based particle size distribution measured using a laser diffraction granulometer.

In the case where the carbon black-containing composite particles have the shape of a flake, the average particle diameter of the carbon black-containing composite particles is, for example, 1 $\mu$m to 100 $\mu$m, desirably 2 $\mu$m to 50 $\mu$m, and more desirably 3 $\mu$m to 40 $\mu$m. The thickness of the carbon black-containing composite particles is, for example, 0.1 $\mu$m to 5 $\mu$m, desirably 0.1 $\mu$m to 3 $\mu$m, and more desirably 0.1 $\mu$m to 2 $\mu$m. Thus, the coating film in which the carbon black-containing composite particles are dispersed has a good hiding property more reliably.

The content of the carbon black particles in the carbon black-containing composite particles is, for example, 1 mass% to 80 mass%, desirably 10 mass% to 70 mass%, and more desirably 20 mass% to 60 mass%. This makes it likely that the carbon black particles in the carbon black-containing composite particles are appropriately dispersed. Thus, the coating film in which the carbon black-containing composite particles are dispersed has a good hiding property more reliably and the undertone can be reduced advantageously. Meanwhile, the content of the silicon oxide in the carbon black-containing composite particles is, for example, 20 mass% to 99 mass%, desirably 30 mass% to 90 mass%, and more desirably 40 mass% to 80 mass%. This makes it likely that the carbon black particles in the carbon black-containing composite particles are appropriately dispersed. Thus, the coating film in which the carbon black-containing composite particles are dispersed has a good hiding property more reliably and the undertone can be reduced more advantageously.

The carbon black-containing composite particles, for example, have a porosity of 10% to 60%. This makes it likely that the carbon black in the carbon black-containing composite particles is appropriately dispersed, which enables the undertone to be reduced more advantageously. The porosity of the carbon black-containing composite particles can be measured by, for example, the BET method (nitrogen adsorption method).

The carbon black-containing composite particles have great versatility and can be used for various purposes. For example, the carbon black-containing composite particles can be used as a black colorant for a cosmetic, paint, and plastic material. Therefore, a cosmetic of the present invention contains the carbon black-containing composite particles. A paint of the present invention contains the carbon black-containing composite particles. A plastic material of the present invention contains the carbon black-containing composite particles.

Examples of the cosmetic containing the carbon black-containing composite particles include an eyebrow pencil, eyeliner, and mascara. Other than the carbon black-containing composite particles, conventionally known components can be used as components contained in the cosmetic.

The paint containing the carbon black-containing composite particles can be prepared by mixing the carbon black-containing composite particles directly with a matrix for a paint or mixing a dispersion containing the carbon black-containing composite particles with a matrix for a paint. In this case, the dispersion containing the carbon black-containing composite particles is prepared, for example, by dispersing the carbon black-containing composite particles in an organic solvent such as an alcohol, ether, ester, or ketone, for example, methanol, ethanol, n-propanol, isopropanol, butanol, ethylene glycol monomethyl acetate, methyl ethyl ketone, methyl isobutyl ketone, isophorone, toluene, or xylene. As the matrix for a paint, a conventionally known thermosetting resin or thermoplastic resin can be used. For example, a conventionally known polyester resin, polycarbonate resin, polyamide resin, polyphenylene oxide resin, thermoplastic acrylic resin, vinyl chloride resin, fluorine resin, vinyl acetate resin, silicone rubber, urethane resin, melamine resin, silicon resin, butyral resin, reactive silicone resin, phenolic resin, epoxy resin, unsaturated polyester resin, thermosetting acrylic resin, urethane acrylate resin, and epoxy acrylate resin can be used.

The plastic material containing the carbon black-containing composite particles can be produced, for example, by melting a pellet-shaped matrix resin mixed with the carbon black-containing composite particles and then kneading the mixture. As the matrix resin, for example, polyethylene, polypropylene, polyamide, polyacetal, polybutylene terephthalate, polyethylene terephthalate, polyphenylene sulfide, polyvinyl chloride, polystyrene, ABS resin, AS resin, methacryl resin, and polycarbonate can be used. The plastic material containing the carbon black-containing composite particles is, for example, in the form of pellets.

Hereinafter, an example of a method for producing the carbon black-containing composite particles will be described. First, a dispersion containing colloidal silicon oxide particles and carbon black particles is prepared. The dispersion can be prepared, for example, by mixing a dispersion of the colloidal silicon oxide particles and a dispersion of the carbon black particles. The particle diameter of the colloidal silicon oxide particles is, for example, but not particularly limited to, 3 nm to 100 nm. As the dispersion of the colloidal silicon oxide particles, for example, SILICADOL (SILICADOL 30S, SILICADOL 20, SILICADOL 30, SILICADOL N, SILICADOL 20L, or SILICADOL OL) manufactured by Nippon Chemical Industrial Co., Ltd. or SNOWTEX (SNOWTEX XS, SNOWTEX N, SNOWTEX O, SNOWTEX 30, SNOWTEX 20L, or SNOWTEX ZL) manufactured by Nissan Chemical Industries, Ltd. can be used. Next, spray drying is carried out using the dispersion containing the colloidal silicon oxide particles and carbon black particles. Carbon black-containing composite particles having the shape of a sphere can be produced in this manner.

Another example of the method for producing the carbon black-containing composite particles will be described. First, a dispersion containing colloidal silicon oxide particles and carbon black particles dispersed in water and having a pH of 7 or more, is prepared. The particle diameter of the colloidal silicon oxide particles is, for example, but not particularly limited to, 3 nm to 100 nm. Next, this dispersion is introduced into a liquid containing a solvent to yield, in the liquid, aggregates of the colloidal silicon oxide particles in which the carbon black particles are dispersed. The solvent is a protic solvent having a relative permittivity of 30 or less at 20°C or an aprotic solvent having a relative permittivity of 40 or less at 20°C. The solvent has a solubility of 0.05 g/100 ml or more in 20°C water. Then, the aggregates of the colloidal silicon oxide particles in which the carbon black particles are dispersed is subjected to at least one treatment selected from drying, heating, and pressurization to obtain carbon black-containing composite particles insoluble in water.

The solubility of the solvent contained in the liquid is desirably 2 g/100 ml or more in 20°C water. The solvent contained in the liquid is, for example, a solvent miscible with 20°C water (that is, having infinite solubility). In this case, carbon black-containing composite particles having the shape of a flake are likely to be obtained. The solvent contained in the liquid may be a solvent having a solubility of 2 g/100 ml to 100 g/100 ml in 20°C water. In this case, carbon black-containing composite particles having the shape of a sphere are likely to be obtained.

The dispersion containing the colloidal silicon oxide particles and carbon black particles dispersed in water and having a pH of 7 or more is introduced into the above liquid, for example, dropwise. The above liquid may be under stirring during this period of time. Carbon black-containing composite particles having the shape of a flake are likely to be obtained in this manner.

The aggregates of the colloidal silicon oxide particles in which the carbon black particles are dispersed are desirably subjected to drying treatment. In the treatment, the colloidal silicon oxide particles included in the aggregates bind each other to form the carbon black-containing composite particles insoluble in water.

In this case, the aggregates are separated from the liquid before the drying treatment. The separation can be performed by employing a commonly-known solid-liquid separation process such as filtration, centrifugation, or decantation. The aggregates may be washed after the separation from the liquid. For washing the aggregates, for example, an organic solvent having a low molecular weight and boiling point less than 100°C, such as ethanol or acetone, can be used. Conditions of the drying treatment of the aggregates are not particularly limited. The drying treatment may be performed by natural drying (air drying at normal temperature) and is desirably performed in an atmosphere having a temperature of 40°C to 250°C, particularly 50°C to 250°C.

The carbon black-containing composite particles insoluble in water can also be obtained by subjecting the aggregates to heating treatment and/or pressurization treatment. For example, the liquid containing the aggregates is heated and/or pressurized as it is, or heating and/or pressurization is performed after the liquid is replaced with another solvent (a solvent for heating treatment) to obtain the carbon black-containing composite particles insoluble in water.

The heating temperature in the heating treatment is desirably 50°C or higher, and more desirably 70°C or higher, and is, for example, 78 to 85°C. The heating is carried out at a temperature lower than or equal to the boiling point of the liquid. Therefore, especially when the solvent contained in the liquid has a boiling point as low as 50°C or lower, the liquid is desirably replaced with a solvent for heating treatment having a higher boiling point of, for example, 70°C or higher before the heating. The heating time is not particularly limited, and may be set as appropriate depending on the applied temperature or the like. For example, the heating time is 0.1 to 12 hours, and particularly 2 to 8 hours.

The pressure in pressurization treatment is desirably 0.11 MPa or higher, more desirably 0.12 MPa or higher, and particularly desirably 0.13 MPa or higher, and is, for example, 0.12 to 0.20 MPa. For example, the pressurization can be performed by putting the liquid containing the aggregates in a container and setting the pressure of an atmosphere in contact with the liquid at a value as indicated above. The pressurization time is not particularly limited, and may be set as appropriate depending on the applied pressure or the like. For example, the pressurization time is 0.2 to 10 hours, and particularly 1 to 5 hours. In view of brittleness of the aggregates, the pressurization is desirably performed at a static pressure. The heating and pressurization treatment may be carried out simultaneously or sequentially.

Yet another example of the method for producing the carbon black-containing composite particles will be described. First, a dispersion containing colloidal silicon oxide particles and carbon black particles is prepared. The dispersion can be prepared by mixing, for example, a dispersion of the colloidal silicon oxide particles and a dispersion of the carbon black particles. The particle diameter of the colloidal silicon oxide particles is, for example, but not particularly limited to, 3 nm to 100 nm. Then, a predetermined water-soluble substance is added into the dispersion containing the colloidal silicon oxide particles and carbon black particles, and the resultant mixture is stirred to prepare a coating liquid.

Examples of the water-soluble substance include: (i) naturally occurring polysaccharides such as starch, glycogen, cellulose, and saccharose; (ii) semisynthetic water-soluble polymers such as carboxymethyl cellulose, and hydroxyethyl cellulose; and (iii) synthetic water-soluble polymers such as polyvinyl alcohol, polyethylene oxide, and polyethylene glycol.

Next, the coating liquid is applied onto a predetermined support. The support is, for example, a roll-shaped, belt-shaped, or sheet-shaped support. The support is, for example, a roll-shaped support made of a metal such as iron or an alloy such as stainless steel, a chromium-plated roll-shaped support made of a metal such as iron or an alloy such as stainless steel, a roll-shaped support made of a ceramic containing aluminum oxide or zirconium oxide or glass, or a roll-shaped support made of a ceramic containing aluminum oxide or zirconium oxide or glass coated with a polymer such as a silicone rubber. Also, the support is, for example, a belt-shaped or sheet-shaped support made of a metal such as iron, alloy such as stainless steel, ceramic containing aluminum oxide or zirconium oxide, glass, or resin composition such as polyethylene terephthalate (PET), polyimide, or polyamide.

The method for applying the coating liquid to the support is, for example, but not particularly limited to, dipping, bar coating, roll coating, curtain coating, or spraying.

Next, the coating liquid is dried by heating the support or coating liquid to form a thin film. This is followed by wetting the thin film. The wetted thin film is subsequently crushed and exfoliated from the support by rubbing with a soft substance such as a felt, sponge, or urethane foam. Carbon black-containing composite particles having the shape of a flake can be obtained in this manner.

The temperature at which the coating liquid is dried is, for example, but not particularly limited to, 80°C to 250°C. The method for wetting the thin film is, for example, a method in which the thin film is immersed in water along with the support, a method in which water or water vapor is sprayed or jetted onto the thin film using a spray or nozzle, or a method in which the thin film is exposed to a humid atmosphere along with the support.

EXAMPLES

The present invention will be described in detail using Examples. It should be noted that the present invention is not limited to Examples given below. First of all, methods for evaluating properties of carbon black-containing composite particles according to Examples, coating films according to Examples, and a coating film according to Comparative Example will be described.

<Average particle diameter>

The average particle diameter of the carbon black-containing composite particles according to each Example was measured with a laser diffraction-scattering particle size distribution analyzer (manufactured by NIKKISO CO., LTD, product name: Microtrac MT-3000 II). The results are shown in Table 1.

<Thickness of composite particles in the shape of a flake>

The carbon black-containing composite particles according to Examples 2 to 4 were observed with an electron microscope to determine the thickness of the carbon black-containing composite particles in the shape of a flake. The thickness was determined by averaging maximum thickness values of 100 or more composite particles in the shape of a flake. The results are shown in Table 1.

<Porosity>

The porosity of the carbon black-containing composite particles according to each Example was determined by the BET method (nitrogen adsorption method). The results are shown in Table 1.

<Hiding property and chroma>

Using a chroma meter (manufactured by KONICA MINOLTA, INC., product name: CR-300), the lightness ($L_W^*$ and $L_B^*$) was measured for the coating films according to Examples and Comparative Example 1 each formed on the black part and white part of a hiding-chart meeting the standards specified in JIS K 5600-4-1: 1999 and the chroma ($C^*$) was measured for the coating films according to Examples and Comparative Example 1 each formed on the white part of the hiding-chart. Table 1 shows the results for the hiding property $L_W^* - L_B^*$ of the coating films according to Examples and Comparative Example and the chroma $C^*$ of the coating films according to Examples and Comparative Example each formed on the white part of the hiding-chart.

<Example 1>

An amount of 66 parts by mass of a dispersion (manufactured by Nippon Chemical Industrial Co., Ltd., product name: SILICADOL 30S) of colloidal silicon oxide particles and 34 parts by mass of a dispersion (manufactured by Daito Kasei Kogyo Co., Ltd., product name: WD-CB2) of carbon black particles were mixed to prepare a dispersion containing the colloidal silicon oxide particles and carbon black particles. SILICADOL 30S is a colloidal silica containing water as a dispersion medium and having a pH of 9.0 to 10.5, and the particle diameter of the colloidal particles contained therein is 5 to 15 nm. WD-CB2 is a dispersion of LC 902, carbon black particles manufactured by Sensient Technologies Japan Co., Ltd. The concentration of the carbon black particles in the solids content of the dispersion was 30 mass%. The pH of the resultant dispersion containing the colloidal silicon oxide particles and carbon black particles was 10.0. Then, the dispersion containing the colloidal silicon oxide particles and carbon black particles was spray-dried using a spray dryer (manufactured by Nihon BUCHI K.K., product name: Mini Spray Dryer B-290) to produce carbon black-containing composite particles according to Example 1.

The carbon black-containing composite particles according to Example 1 and Auto Clear Super (manufactured by NIPPON PAINT Co., Ltd.) were weighed to a total of 10 g in a 20 ml glass vial, and then the contents were stirred and mixed for 2 minutes with a pencil mixer. Subsequently, the mixture of the carbon black-containing composite particles according to Example 1 and Auto Clear Super was further mixed using a paint shaker (manufactured by Nihonseiki Kaisha Ltd.) for 5 minutes to prepare a paint. The paint was applied to the black part and white part of a hiding-chart meeting the standards specified in JIS K 5600-4-1: 1999 using a 9-mil applicator and dried to form a coating film according to Example 1. The thickness of the coating film was about 31 $\mu$m. The carbon black-containing composite particles according to Example 1 and Auto Clear Super were mixed so that the concentration of the carbon black particles in the coating film would be 1 mass%.

<Example 2>

A dispersion containing colloidal silicon oxide particles and carbon black particles was prepared in the same manner as in Example 1. A volume of 50 ml of 2-propanol (isopropyl alcohol) was put in a beaker, and a total amount of 1 g of the dispersion containing the colloidal silicon oxide particles and carbon black particles and prepared in the same manner as in Example 1 was added in drops of 0.01 g. During the dropping of the silica sol, the 2-propanol was stirred with a magnetic stirrer (rotational speed: 800 rpm). Next, aggregates of the colloidal silicon oxide particles were separated by centrifugation from the solvent in which the colloidal silicon oxide particles had been aggregated to form a slurry. The aggregates were washed with 2-propanol, which was then removed by decantation. The obtained aggregates of the colloidal particles were dried in a vacuum dryer set at 150°C to obtain carbon black-containing composite particles according to Example 2. The carbon black-containing composite particles according to Example 2 had the shape of a flake. 2-Propanol is a protic solvent, has a relative permittivity of 18 at 20°C, and is miscible with 20°C water.

A coating film according to Example 2 was formed in the same manner as in Example 1, except for using the carbon black-containing composite particles according to Example 2 instead of the carbon black-containing composite particles according to Example 1. The thickness of the coating film according to Example 2 was about 30 $\mu$m.

<Example 3>

A dispersion containing colloidal silicon oxide particles and carbon black particles was prepared in the same manner as in Example 1. In this dispersion, an amount of saccharose (molecular weight: 342) corresponding to 10 weight% of the total solid weight of the dispersion was added. The resultant mixture was then stirred for 30 minutes to prepare a coating liquid. This coating liquid was applied to a PET film having a width of 10 cm using a bar coater so that the thickness of a thin film to be obtained after drying would be 1.0 $\mu$m. Next, the PET film to which the coating liquid had been applied was placed in a drying oven set at 120°C for 3 minutes to dry the coating liquid. The PET film was taken out of the drying oven and cooled to room temperature. At that time, the thin film was uniformly adhered onto the PET film surface. While being sprayed with room-temperature water, the PET film alone was pulled to move relative to a felt held with a commercially-available binder clip closely onto the surface where the thin film had been formed. This exfoliated the thin film from the PET film surface. The area of the PET film from which the thin film was exfoliated was measured to be 90% or larger. The exfoliated thin film scales were collected and dried at 120°C for 1 hour. In this manner, carbon black-containing composite particles according to Example 3 were obtained. The carbon black-containing composite particles according to Example 3 had the shape of a flake.

A coating film according to Example 3 was formed in the same manner as in Example 1, except for using the carbon black-containing composite particles according to Example 3 instead of the carbon black-containing composite particles according to Example 1. The thickness of the coating film according to Example 3 was about 36 $\mu$m.

<Example 4>

A dispersion containing colloidal silicon oxide particles and carbon black particles was prepared in the same manner as in Example 1, except for mixing 72 parts by mass of a dispersion (manufactured by Nippon Chemical Industrial Co., Ltd., product name: SILICADOL 30S) of the colloidal silicon oxide particles and 28 parts by mass of a dispersion (Action black A90 manufactured by Harold Scholz & Co. GmbH) of the carbon black particles instead of mixing 66 parts by mass of the dispersion of the colloidal silicon oxide particles and 34 parts by mass of the dispersion (manufactured by Daito Kasei Kogyo Co., Ltd., product name: WD-CB2) of the carbon black particles. The concentration of the carbon black particles in the solids content of the dispersion was 30 mass%. Action black A90 is a dispersion of carbon black (Raven 1225) manufactured by Birla Carbon. Except for using this dispersion, carbon black-containing composite particles according to Example 4 were obtained in the same manner as in Example 2. The carbon black-containing composite particles according to Example 4 had the shape of a flake.

A coating film according to Example 4 was formed in the same manner as in Example 1, except for using the carbon black-containing composite particles according to Example 4 instead of the carbon black-containing composite particles according to Example 1. The thickness of the coating film according to Example 4 was about 30 μm.

<Example 5>

A dispersion containing colloidal silicon oxide particles and carbon black particles was prepared in the same manner as in Example 1. A volume of 50 ml of 2-methyl-1-propanol was put in a beaker, and a total amount of 1 g of the dispersion containing the colloidal silicon oxide particles and carbon black particles and prepared in the same manner as in Example 1 was added in drops of 0.01 g. During the dropping of the silica sol, the 2-methyl-1-propanol was stirred with a magnetic stirrer (rotational speed: 800 rpm). Next, aggregates of the colloidal silicon oxide particles were separated by centrifugation from the solvent in which the colloidal silicon oxide particles had been aggregated to form a slurry. The aggregates were washed with 2-methyl-1-propanol, which was then removed by decantation. The obtained aggregates of the colloidal particles were dried in a vacuum dryer set at 150°C to obtain carbon black-containing composite particles according to Example 5. The carbon black-containing composite particles according to Example 5 had the shape of a sphere.

A coating film according to Example 5 was formed in the same manner as in Example 1, except for using the carbon black-containing composite particles according to Example 5 instead of the carbon black-containing composite particles according to Example 1. The thickness of the coating film according to Example 5 was about 32 μm.

<Comparative Example 1>

A coating film according to Comparative Example 1 was formed in the same manner as in Example 1, except for using LC 902 carbon black particles manufactured by Sensient Technologies Japan Co., Ltd. instead of the carbon black-containing composite particles according to Example 1. The thickness of the coating film according to Comparative Example 1 was about 29 μm.

The carbon black-containing composite particles according to Examples 1 to 5 were dispersed in Auto Clear Super easily and uniformly. The coating films according to Examples 1 to 5 showed a superior hiding property compared to the coating film according to Comparative Example 1. Especially the coating films according to Examples 2 and 4 showed a superior hiding property. This is presumably because the carbon black-containing composite particles according to Examples 2 and 4 were likely to be arranged in parallel in the coating films to form a large projected area due to their thickness on the order of submicrons. The paints according to Examples 2 and 4, which contained different kinds of carbon black, showed similar characteristics. The chroma C* of the coating films according to Examples 1 to 5 formed on the white parts of the hiding-charts was lower than that of the coating film according to Comparative Example 1 formed on the white part of the hiding-chart. Especially the chroma C of the coating film according to Example 1 was low. This is presumably because the aggregates of the carbon black particles were bound together in the carbon black-containing composite particles according to Example 1.

[Table 1]

| | Shape of composite particles | Content of carbon black in composite particles | Average particle diameter [μm] | Thickness of composite particles [μm] | Porosity | Hiding property $L_w^*$ - $L_B^*$ of coating film | Chroma C* of coating film on white part of hiding-chart |
|---|---|---|---|---|---|---|---|
| Example 1 | Sphere | 30 mass% | 4.4 | - | 22% | 26.0 | 1.1 |

(continued)

|  | Shape of composite particles | Content of carbon black in composite particles | Average particle diameter [$\mu$m] | Thickness of composite particles [$\mu$m] | Porosity | Hiding property $L_W^*$ - $L_B^*$ of coating film | Chroma C* of coating film on white part of hiding-chart |
|---|---|---|---|---|---|---|---|
| Example 2 | Flake | 30 mass% | 19.9 | 0.8 | 48% | 12.7 | 3.3 |
| Example 3 | Flake | 30 mass% | 10.1 | 1.1 | 29% | 20.7 | 1.7 |
| Example 4 | Flake | 30 mass% | 17.5 | 0.8 | 51% | 13.9 | 3.9 |
| Example 5 | Sphere | 30 mass% | 5.4 | - | 30% | 21.3 | 2.5 |
| Comparative Example 1 | - | - | - | - | - | 43.4 | 5.6 |

**Claims**

1. Carbon black-containing composite particles each comprising a silicon oxide and carbon black particles dispersed inside the silicon oxide, wherein
when a coating film containing the carbon black-containing composite particles dispersed in a transparent matrix, having a concentration of the carbon black particles of 1 mass%, and having a thickness of 25 $\mu$m to 40 $\mu$m is formed on a hiding-chart specified in JIS (Japanese Industrial Standards) K 5600-4-1: 1999, a difference $L_W^* - L_B^*$ obtained by subtracting lightness $L_B^*$ of the coating film formed on a black part of the hiding-chart from lightness $L_W^*$ of the coating film formed on a white part of the hiding-chart is 30 or less.

2. The carbon black-containing composite particles according to claim 1, wherein chroma C* of the coating film formed on the white part of the hiding-chart is 5 or less.

3. The carbon black-containing composite particles according to claim 1, each having the shape of a sphere or a flake.

4. The carbon black-containing composite particles according to claim 2, each having the shape of a sphere or a flake.

5. A cosmetic comprising the carbon black-containing composite particles according to any one of claims 1 to 4.

6. A paint comprising the carbon black-containing composite particles according to any one of claims 1 to 4.

7. A plastic material comprising the carbon black-containing composite particles according to any one of claims 1 to 4.

8. A method for producing the carbon black-containing composite particles according to any one of claims 1 to 4, comprising:

introducing a dispersion containing colloidal silicon oxide particles and carbon black particles dispersed in water and having a pH of 7 or more into a liquid containing a solvent to yield, in the liquid, aggregates of the colloidal silicon oxide particles in which the carbon black particles are dispersed, the solvent being a protic solvent having a relative permittivity of 30 or less at 20°C or an aprotic solvent having a relative permittivity of 40 or less at 20°C, the solvent having a solubility of 0.05 g/100 ml or more in 20°C water; and
subjecting the aggregates to at least one treatment selected from drying, heating, and pressurization to obtain the carbon black-containing composite particles insoluble in water.

**INTERNATIONAL SEARCH REPORT**

| International application No. |
| --- |
| PCT/JP2016/004446 |

| A. CLASSIFICATION OF SUBJECT MATTER |
| --- |
| C09C1/48(2006.01)i, A61K8/19(2006.01)i, A61K8/25(2006.01)i, A61Q1/02 (2006.01)i, C09C1/58(2006.01)i, C09C3/06(2006.01)i, C09D7/12(2006.01)i, C09D201/00(2006.01)i |
| According to International Patent Classification (IPC) or to both national classification and IPC |

| B. FIELDS SEARCHED |
| --- |
| Minimum documentation searched (classification system followed by classification symbols) |
| C09C1/48, A61K8/19, A61K8/25, A61Q1/02, C09C1/58, C09C3/06, C09D7/12, C09D201/00 |

| Documentation searched other than minimum documentation to the extent that such documents are included in the fields searched |
| --- |
| Jitsuyo Shinan Koho 1922–1996 Jitsuyo Shinan Toroku Koho 1996–2016 Kokai Jitsuyo Shinan Koho 1971–2016 Toroku Jitsuyo Shinan Koho 1994–2016 |

| Electronic data base consulted during the international search (name of data base and, where practicable, search terms used) |
| --- |
| |

| C. DOCUMENTS CONSIDERED TO BE RELEVANT |
| --- |

| Category* | Citation of document, with indication, where appropriate, of the relevant passages | Relevant to claim No. |
| --- | --- | --- |
| X A | JP 8-259838 A (Dainippon Printing Co., Ltd.), 08 October 1996 (08.10.1996), claims; paragraphs [0003], [0007] to [0011]; examples (Family: none) | 1-7 8 |
| X A | JP 2004-224950 A (Toyo Ink Manufacturing Co., Ltd.), 12 August 2004 (12.08.2004), claims; paragraphs [0007], [0034] to [0035], [0040]; examples (Family: none) | 1-7 8 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 13 December 2016 (13.12.16) | 20 December 2016 (20.12.16) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/004446

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 9-202862 A (Catalysts & Chemicals Industries Co., Ltd.),<br>05 August 1997 (05.08.1997),<br>claims; paragraphs [0005], [0018]; examples<br>(Family: none) | 1-7<br>8 |
| X<br>A | JP 8-277347 A (The Yokohama Rubber Co., Ltd.),<br>22 October 1996 (22.10.1996),<br>claims; paragraphs [0005], [0007]; examples<br>& US 5679728 A<br>claims; column 1, line 65 to column 2, line 21;<br>column 2, lines 33 to 46; examples<br>& EP 711805 A1        & DE 69516635 D | 1-7<br>8 |
| X<br>A | JP 2003-327867 A (Toda Kogyo Corp.),<br>19 November 2003 (19.11.2003),<br>claims; paragraphs [0002], [0016], [0049], [0059]; examples<br>(Family: none) | 1-7<br>8 |
| A | JP 6-210631 A (NOK Corp.),<br>02 August 1994 (02.08.1994),<br>entire text<br>(Family: none) | 1-8 |
| A | JP 3-002280 A (Degussa AG.),<br>08 January 1991 (08.01.1991),<br>entire text<br>& US 5008223 A<br>entire text<br>& EP 385247 A1        & DE 3906818 A<br>& BR 9001022 A        & CS 9000979 A | 1-8 |
| A | JP 11-100525 A (The Yokohama Rubber Co., Ltd.),<br>13 April 1999 (13.04.1999),<br>entire text<br>& US 2001/0009654 A1<br>entire text<br>& WO 1998/049241 A1      & EP 955343 A1<br>& KR 10-2000-0022337 A | 1-8 |
| A | JP 2002-265815 A (Mitsubishi Chemical Corp.),<br>18 September 2002 (18.09.2002),<br>entire text<br>(Family: none) | 1-8 |

Form PCT/ISA/210 (continuation of second sheet) (January 2015)

**EP 3 360 932 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6136287 A
- JP 2002371219 A
- JP 2004204175 A